# EUROPEAN PATENT APPLICATION

(11) **EP 4 796 047 A1**
(43) Date of publication of application: **26.08.2026**
(21) Application number: 25382170.6
(22) Date of filing: 25.02.2025
(51) Int. Cl.: A61B 8/08, G06T 7/00, G06T 7/246

(54) **SYSTEM AND METHOD FOR DETECTION AND SEGMENTATION OF VEGETATIONS IN ECHOCARDIOGRAPHIC IMAGES**

(71) Applicant: Gerencia Regional de Salud de Castilla y León, 47007 Valladolid (ES); Consorcio Centro de Investigación Biomédica en Red, 28029 Madrid (ES); Fundación Inst. de Est. de Ciencias de la Salud de C. y León (IECSCYL), 42002 Soria (ES)
(72) Inventor: San Román Calvar, José Alberto, Valladolid (ES); Pinilla García, Daniel, Valladolid (ES); Baladrón Zorita, Carlos, Madrid (ES); López Díaz, Javier, Valladolid (ES); Gómez Salvador, Itziar, Soria (ES); Carrasco Moraleja, Manuel, Soria (ES)
(74) Representative: Pons IP

(57) **Abstract**

System for detecting vegetations inside the heart through the analysis of echocardiographic images, comprising a detection module configured to receive echocardiographic images and to detect one or more vegetations in each echocardiographic image using a deep neural network trained with labelled echocardiographic images; a segmentation module configured to determine a contour of the vegetations in the echocardiographic images; and a parameter extraction module configured to analyse sequential echocardiographic images in order to determine position and motion parameters of the vegetations, such as vegetation area, position, velocity, and trajectory. The system further enables accurate diagnosis of embolism risk and the detection of heart valve obstructions. The invention also relates to a method for detecting vegetations that makes use of the described system.

## Description

### OBJECT OF THE INVENTION

The present invention falls within the field of computer vision algorithms, and more specifically, within the application of such algorithms in the medical field.

The objective of the present invention is to develop a system for detecting vegetations inside the heart, which enables the accurate identification of vegetations within the heart enhancing diagnostic capabilities.

Another object of the present invention is a method for detecting vegetations that uses said system and allows for the automation of the determination, measurement, and characterization of vegetations through computer vision algorithms.

### BACKGROUND OF THE INVENTION

Infective endocarditis (IE) is a potentially life-threatening condition characterized by infection of the endocardial surface of the heart, often leading to the formation of vegetations on the heart valves. These vegetations are mobile masses composed of platelets, fibrin, microbial microcolonies, and a few inflammatory cells.

One of the serious complications associated with IE is the occurrence of embolic events, which can lead to stroke, organ infarction, and other significant morbidities. Despite advances in medical and surgical therapies, the mortality rate for patients with IE remains high. Embolic risk in IE patients is a critical factor in clinical decision-making and in improving patient outcomes.

Currently, the measurement of vegetation parameters is performed manually by a human operator, who draws lines or points on an image and, in the case of movement, subjectively and qualitatively assesses the mobility of the vegetation.

The lack of standardized criteria and protocols for performing these assessments results in high intra- and inter-observer variability when making measurements that can determine the patient's optimal treatment. Moreover, this variability leads to inconsistent data and unreliable predictions.

Current scientific literature shows a relationship between vegetation parameters and disease outcomes, with one of the main reasons being the potential of these vegetations to cause embolic events. Therefore, current clinical practice guidelines include the measurement of vegetation diameter as one of the key parameters in deciding patient treatment, as this size is indicative of disease severity and thrombotic potential. Some authors have even proposed risk calculators for embolic events based on multiparametric evaluation, considering clinical and microbiological factors, vegetation size, and, in certain cases, a qualitative estimation of vegetation mobility.

However, these current solutions suffer from several limitations. First, inter-observer variability in measuring vegetation diameter has been shown to be very high, which means that the same patient could receive very different treatment recommendations depending on the echocardiographer performing the measurement. And second, the published models have demonstrated very limited predictive capability.

On the other hand, Artificial Intelligence (AI) has been shown to improve diagnosis in various types of medical conditions, achieving remarkable results in many areas where it can even rival expert clinicians.

Computer vision techniques based on artificial intelligence have been employed successfully in the detection of breast cancer, meningiomas, gliomas, and pituitary brain tumors, in the detection and classification of various dental features from X-ray images, and several applications in the field of echocardiographic imaging.

However, currently, there is no system capable of automatically detecting and segmenting vegetations inside the heart. Nor are there tools available that can automatically extract movement and structural parameters of these vegetations.

### DESCRIPTION OF THE INVENTION

The present invention focuses on an automated and reliable system for the detection and segmentation of vegetations inside the heart through the analysis of echocardiographic images, preferably transoesophageal echocardiographic images.

The system of the invention receives as an input a sequence of echocardiographic images, preferably transoesophageal, and performs the following three operations:
1. Detects the presence of a vegetation in each image or video frame.
2. Segments the vegetation by outlining its contour in each image or video frame.
3. Preferably, also extracts parameters such as position, diameter, area, trajectory, and velocity of the vegetation in each image or video frame.

The system not only automates the diagnosis of vegetations but also extracts parameters that enhance the robustness and consistency of current vegetation size measurements. Additionally, it can also extract previously unstudied parameters, such as the trajectory and velocity of the vegetation in each image or video frame. This enables a more accurate prognosis and risk assessment for each patient with infective endocarditis, thereby improving their management and treatment.

The system of the invention has demonstrated high accuracy in identifying vegetations, representing a significant advancement over traditional manual detection methods, which are often subject to operator variability. By providing consistent and reliable measurements, the system has the potential to serve as a valuable tool for physicians in the diagnosis and treatment of infective endocarditis. Moreover, the ability to automatically detect and measure vegetations is crucial for assessing the risk of embolic events and making informed clinical decisions.

The system of the invention comprises:
- A detection module configured to receive echocardiographic images and detect one or more vegetations in each echocardiographic image using a deep neural network trained with labelled echocardiographic images; and
- A segmentation module connected to the detection module and configured to receive one or more echocardiographic images in which one or more vegetations have been detected by the detection module, and configured to determine the contour of the vegetations in the echocardiographic images.

The system may also include a parameter extraction module connected to the segmentation module, configured to receive one or more segmented echocardiographic images from the segmentation module and to analyse the sequence of echocardiographic images to determine parameters such as shape, size, position, and movement of the vegetations.

The system of the invention may further include a diagnostic module configured to provide a diagnostic value associated with the risk of embolism or death based on the position and movement parameters of the vegetations, in addition to the patient's clinical variables.

The system may also include an initial preprocessing module configured to extract echocardiographic images from an echocardiographic video.

This preprocessing module may also be configured to apply the following operations to each echocardiographic image:
- Extract numerical values of the intensity of each pixel;
- Convert the echocardiographic image to grayscale;
- Delimit an echocardiographic cone; and
- Set to zero all pixels outside the echocardiographic cone.

The detection module may also be configured to detect heart structures, and the segmentation module to determine an overlapping region between the vegetations and the internal structures of the heart.

The system may further include a visualization module configured to generate a visual representation of the segmented vegetations and/or the calculated parameters.

Such parameters may be selected from: trajectory, acceleration, area, velocity, and position.

The detection module may also determine a confidence value for the detection, which can then be used to filter out detections that do not exceed a confidence threshold.

Preferably, the detection module may implement a convolutional-type architecture.

The segmentation module may be configured to determine the contour that encloses each vegetation, determining its centre and dimensions.

In preferred implementations, the segmentation module is trained using segmented images processed by an image processor configured to generate masks.

The parameter extraction module may be configured to calculate the area of the vegetation in each frame by counting the number of pixels occupied by the segmented vegetation and multiplying the number of pixels by the area of each pixel obtained using the metadata of the echocardiographic image.

This parameter extraction module may also calculate the velocity and trajectory of the vegetations by comparing multiple consecutive frames and using a tracking algorithm.

The parameter extraction module may also determine the trajectory starting from a position in the previous frame and adjusting weights assigned to each tracking algorithm.

Additionally, the system of the invention may include a valve monitoring module configured to locate the heart valves in the echocardiographic images using neural networks trained with labelled valve images.

The invention also includes a method for detecting vegetations inside the heart that utilizes the described system, and which comprises the following steps:
- Providing one or more echocardiographic images of the inside of the heart;
- Detecting one or more vegetations in each echocardiographic image using the detection module, employing a specifically trained deep neural network;
- Segmenting each of the echocardiographic images in which vegetations have been detected to determine the contour of the vegetations using the segmentation module; and
- Extracting position and motion parameters from the segmented images using the parameter extraction module.

Preferably, the step of providing one or more echocardiographic images of the interior of the heart may comprise the following steps:
- acquiring an echocardiographic video; and
- extracting echocardiographic images from the echocardiographic video corresponding to the frames of said echocardiographic video.

Additionally, the method of the invention may also include an initial preprocessing stage including the following steps:
- extracting numerical values of the intensity of each pixel;
- converting the echocardiographic image to grayscale;
- delimiting an echocardiographic cone; and
- masking out all pixels outside the echocardiographic cone.

With regard to the step of segmenting each of the echocardiographic images, said step may further comprise determining a bounding frame that encloses a vegetation, defining its center and its dimensions.

The step of extracting position and motion parameters from the segmented images may further comprise calculating an area of the vegetations by counting the number of pixels occupied by each segmented vegetation and multiplying the number of pixels by the area of each pixel, said area being obtained using metadata from the echocardiographic image.

The step of extracting parameters may also comprise calculating the speed and trajectory of the vegetations by comparing multiple consecutive frames and applying a tracking algorithm.

The method of the present invention may further comprise a step of analysing the confidence of detections and segmentations by comparing the obtained parameters with the same parameters of previously identified vegetations, said comparison being performed by means of time series decomposition models and/or artificial intelligence algorithms for pattern analysis.

Additionally, in the method of the present invention, the presence of valves in the echocardiographic images may be determined by means of neural networks trained with labeled valve images.

### DESCRIPTION OF FIGURES

In order to complete the description being provided and to facilitate a better understanding of the characteristics of the invention, a set of figures is attached as an integral part of said description. The figures are presented for illustrative and non-limiting purposes and depict the key components and workflows of the proposed system.
- Figure 1 shows an example block diagram of the system of the invention for the analysis of an echocardiographic video.
- Figure 2 shows an example execution of the preprocessing stage of the method of the invention over a sample input.
- Figure 3 shows an example execution of the detection stage of the method of the invention over a sample input.
- Figure 4 shows an example execution of the segmentation stage of the method of the invention over a sample input.
- Figures 5A, 5B, 5C, 5D shows an example of parameters obtained during a position and motion parameter extraction stage for vegetations in the method of the invention over a sample input.
- Figure 6 shows an example block diagram of an area calculation step within the parameter extraction stage of the method of the invention.
- Figure 7 shows an example block diagram of a speed and acceleration calculation step within the parameter extraction stage of the method of the invention.
- Figure 8 shows a F1 curve of an exemplary embodiment of the method of the invention applied to a test database with 244 patients.
- Figure 9 shows a precision curve of an exemplary embodiment of the method of the invention applied to a test database with 244 patients.
- Figure 10 shows a recall curve of an exemplary embodiment of the method of the invention applied to a test database with 244 patients.
- Figure 11 shows a ROC curve of an exemplary embodiment of the method of the invention applied to a test database with 244 patients with a threshold over the confidence parameter of 0.1.

### PREFERRED EMBODIMENT OF THE INVENTION

The present invention is a system for the detection of vegetations inside the heart, which enables the automatic detection of vegetations in echocardiographic images.

The system of the invention employs a high-performance algorithm that allows the standardization of measurements across different operators, thereby improving the diagnosis and treatment of infective endocarditis and eliminating both intra-operator and inter-operator variability.

The system of the invention provides high accuracy in the automatic detection of vegetations in patients with infective endocarditis.

Infective endocarditis can lead to intracardiac lesions known as vegetations. The presence of such lesions is one of the main criteria for the definitive diagnosis of the disease. Moreover, current scientific knowledge has established a correlation between the presence and certain characteristics of these vegetations (length, area, mobility) and the risk of embolism and death during the course of the disease.

Furthermore, the system of the invention could be applied not only for the detection of vegetations, but also for the detection of other types of abnormal mobile masses within the heart, such as intracardiac tumours, thrombi, and the like. The system could also be useful for detecting structural abnormalities, such as ruptured chordae, valvular prolapse, and similar conditions. For such applications, the images and labels used for training the system must be adapted accordingly.

The system of the invention receives echocardiographic images and, specifically, may receive images from transoesophageal echocardiography. The images are extracted from video sequences as individual frames.

In certain cases, the neural networks operate on static 2D images, and the results from each frame are subsequently processed in order to integrate information from the entire sequence, with the aim of identifying false detections in individual frames, determining the maximum length or area across the entire sequence, extracting the trajectory of the detected vegetation, and the like.

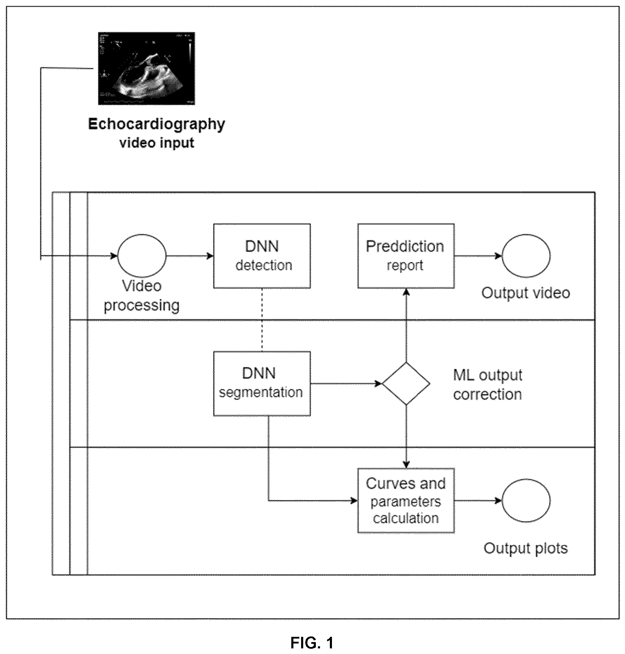

Figure 1 illustrates an exemplary embodiment of the vegetation detection system of the invention. This system comprises:
- a detection module configured to receive echocardiographic images and to detect one or more vegetations in each echocardiographic image using a deep neural network trained with labelled echocardiographic images;
- a segmentation module connected to the detection module and configured to receive one or more echocardiographic images in which one or more vegetations have been detected by the detection module, and configured to determine a contour of the vegetations in the echocardiographic images; and
- a parameter extraction module connected to the segmentation module and configured to receive one or more segmented echocardiographic images from the segmentation module, and to analyse the sequential echocardiographic images in order to determine position and motion parameters of the vegetations.

The parameter extraction module allows the integration of information from different frames over time in order to extract parameters such as length, area, velocity, and trajectory along time.

In addition, in this case, the system further comprises a diagnostic module configured to estimate the risk of embolism or death based on the analysis of the echocardiographic images. The results may also be enhanced with additional clinical data from the patient.

In one exemplary embodiment, a database was compiled comprising 244 patients, including both the images to be processed and clinical data from the patient prior to and during hospitalization, as well as the outcome of the episode.

Both the variables collected in the database and the way in which the data are requested in each of the questionnaires were jointly defined by physicians, statisticians, and engineers. The questionnaire was structured into three main sections: baseline characteristics, clinical evolution, and echocardiographic imaging.

The system also comprises, in this case, a preprocessing module responsible for obtaining an echocardiographic video, extracting echocardiographic images, and cleaning the images using mathematical methods.

Echocardiographic videos are obtained from DICOM files generated during the echocardiogram and anonymized before being used.

The preprocessing module is configured to perform the following steps:
- Extract numerical values corresponding to the pixel intensities of the video and store them in a matrix.
- Standardize the number of video channels to ensure consistency regardless of the echocardiograph used.
- Convert the video from RGB format to grayscale.
- Crop a region of interest from the video using the DICOM file metadata.
- Locate an echocardiographic cone and generate a mask for the inner zone using an Optical Flow algorithm.
- Set to zero all pixels outside the echocardiographic cone.
- Adjust the resolution of the processed videos to match the input format required by the detection module, ensuring that the dimensions are compatible with the neural network specifications. In this case, the input data are grayscale images with dimensions 544x544. The colour values are normalized between 0 and 1, and the data are 64-bit floating-point values where the value is proportional to the image intensity.
- Separate the videos into frames for subsequent processing.

To determine the perimeter points of the echocardiographic cone, optical-flow modules from the OpenCV library can be used, based on the Lucas-Kanade algorithm and the Gunnar Farneback algorithm. A linear regression on these points is used to determine the perimeter of the cone.

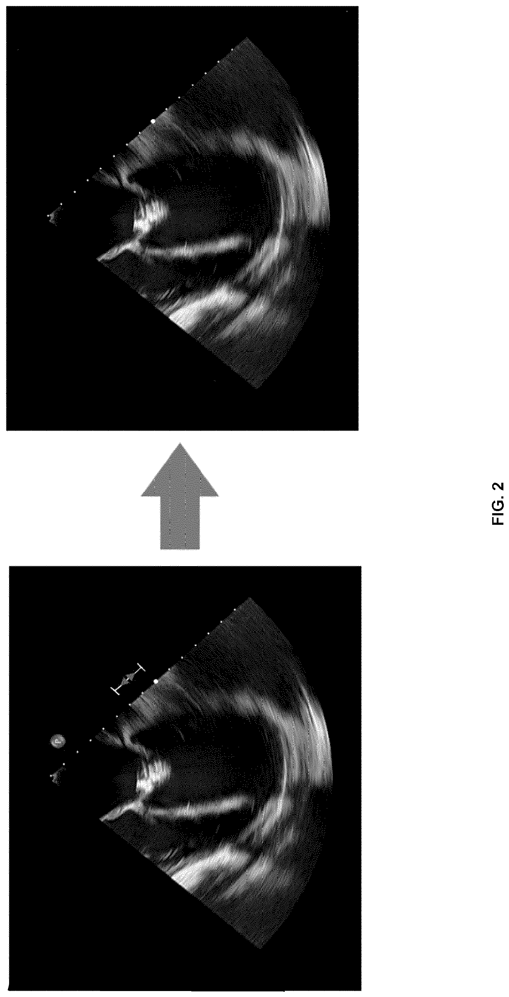

Figure 2 shows an example of the results from the preprocessing stage.

The detection and segmentation modules utilize deep neural networks trained with anonymized images, without pretraining. They may also employ machine learning algorithms such as random forest and k-means clustering to evaluate the consistency of the obtained parameters and discard data considered as outliers. The random forest algorithm used is preferably a variation of the TimeSeriesForestClassifier method from the sktime library. This model has been trained using detection curves generated from video frames, where detection errors have been marked to identify whether a specific data point corresponds to an outlier.

On the other hand, the k-means algorithm complements the functionality of the random forest in identifying outliers. This approach is similar to the KNeighborsTimeSeriesClassifier algorithm from the same library and performs its analysis based on the distance between the parameters detected in one frame and the remaining frames. In this way, both algorithms work together to ensure consistency and robustness in detection and segmentation. In both cases, experiments have been conducted to optimize training hyperparameters.

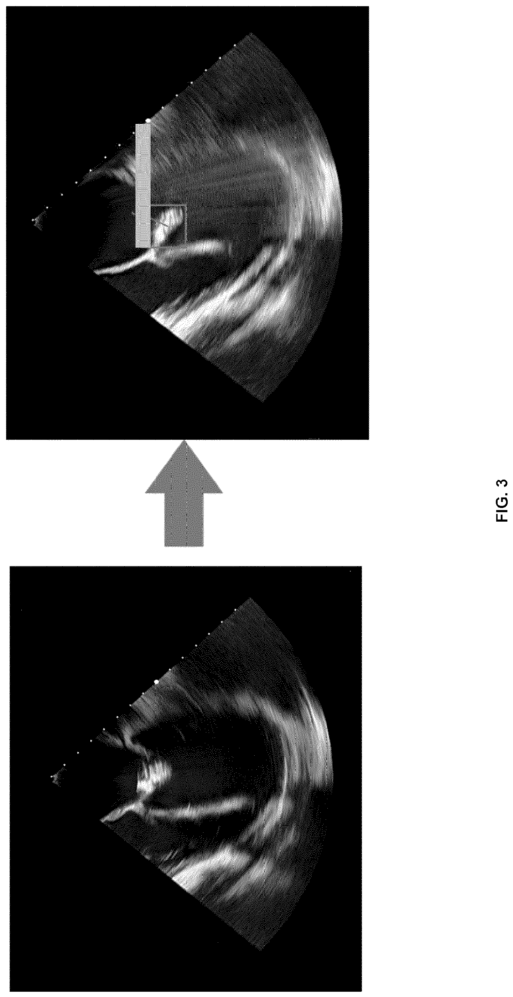

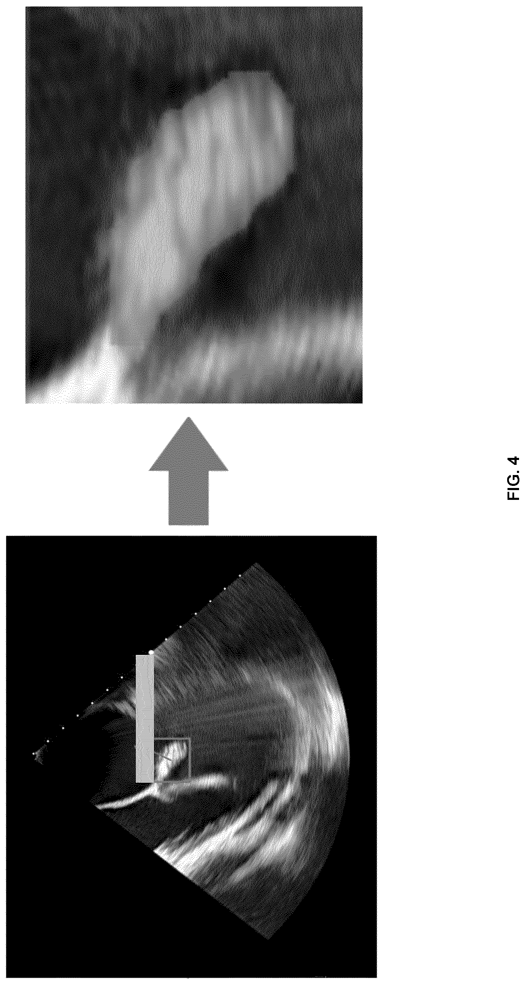

Figures 3 and 4 illustrate an exemplary embodiment of the results obtained from the detection and segmentation stages, respectively, of the method of the invention.

The detection module comprises a deep neural network responsible for detecting vegetations in each frame. Specifically, in this case, it detects whether there is a vegetation in a given frame. If a vegetation is detected, the detection module identifies its position and provides a confidence value for the detection, which is an estimate of the probability that the detected vegetation is indeed a vegetation. In the detection module, a simple filter can be implemented based on a minimum confidence threshold that the detections must exceed in order to be considered detected vegetations. Detections whose confidence value does not exceed this threshold are considered detection errors and are not sent to the segmentation module.

In a particular configuration, the detection module is implemented using an architecture of a real-time object detector, such as Yolov11, with no pretraining, with weights initialized randomly, and trained with a custom database.

The model architecture is organized into three main components, which together include approximately 15 million trainable parameters. The initial stage of the architecture consists of sequential 2D convolution modules and C3K2 modules. Each convolution layer is paired with a normalization layer and uses the SiLU activation function. The C3K2 modules include two convolution blocks at their boundaries, interspersed with CSP blocks.

The data flow begins with an initial convolution block, followed by a sequence of n bottleneck-type layers with concatenations, and concludes with a final convolution block. The results of this process are concatenated with the outputs from the last C3K2 block and then forwarded to an additional convolution block. Each C3K2 module is connected to different branches, which are in turn linked to the second stage.

The intermediate stage acts as a bridge between the initial and output stages, employing a design that aggregates features from multiple image regions at different scales. Convolution modules and C3K2 layers process the outputs from the various branches originating in the initial stage.

To align and concatenate the matrices from these branches, upsampling modules are used. In addition, attention mechanisms have been incorporated into the branch operating at smaller scales, using C2PSA blocks. The intermediate stage generates three branches, each connected to the final stage. The output stage processes feature maps produced by the second stage to estimate the probability of detecting an instance in a specific region of the image. This architecture receives input tensors with dimensions (N, H, W, C), where N is the batch size, H and W represent the height and width of the resized image, and C is the number of channels. As output, the model produces a tensor that includes the coordinates of bounding boxes, along with confidence scores indicating the probability that each bounding box contains an object.

The detection module outputs the coordinates of the centres of a bounding box that encloses the vegetation, along with its width and height.

For the training of the neural network in the detection module, a total of 34,116 transoesophageal echocardiography (TEE) images of the heart were manually processed, generating a collection of square bounding boxes, each containing one of the vegetations present in the image. A Visual Object Tagging Tool (VoTT)-type software was used to label the vegetations in the videos, allowing multiple vegetations to be annotated in the same frame. The training dataset included images from patients both with and without vegetations. Labelling was performed by fitting each vegetation within a bounding box and recording its centre coordinates, width, and height.

The segmentation module is responsible for segmenting the detected vegetations, meaning extracting their exact contour and, in this case, determining their overlapping region with internal heart structures. The segmentation module uses as input the bounding boxes determined by the detection module.

The neural network architecture of the segmentation module is similar to the architecture of the detection module, except for the final stage, which in this case is a set of tuples representing the coordinates of the points that define the contour of the vegetation.

The neural networks of the detection and segmentation modules can employ a set of convolutional filters (CNN) organized in a pyramidal structure, combined with CSP (Cross Stage Partial) blocks.

A Convolutional Neural Network (CNN) is a specialized type of artificial neural network designed to process grid-like data structures, particularly images. CNNs excel at computer vision tasks by using convolutional layers to automatically learn hierarchical features and patterns from input images. These layers apply small filters to perform convolution operations, capturing local entities and spatial hierarchies.

The segmentation module outputs a mask of the vegetation contour over an image with dimensions similar to the input.

The neural network of the segmentation module was trained with images cropped from the bounding boxes defined during the training of the detection module's neural network, in which vegetations had been located. In these images, manual segmentation of the identified vegetations was performed. To regulate the spacing between points, segmentation masks were generated, and the perimeter of each mask was obtained using an image processing script.

The parameter extraction module collects all the vegetations detected in each frame of every video sequence and calculates a set of parameters for each time point.

The parameter extraction module integrates all frames, as the calculation of certain parameters, such as velocity, requires comparisons between consecutive frames.

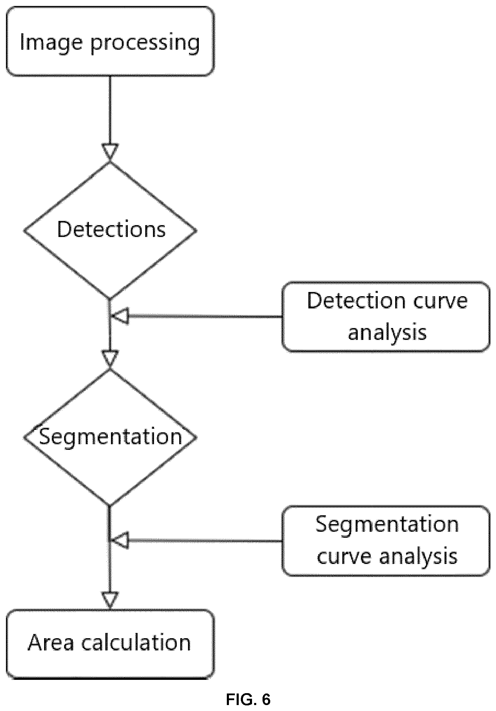

For each time point within each sequence, the parameter extraction module provides the following parameters:
- Area: In each frame, based on the contour of the vegetations obtained from the segmentation module, the number of pixels enclosed within the contour is counted and multiplied by the area of each pixel, which is obtained from the metadata of the DICOM file. Figure 6 shows a schematic example of the area calculation step.
- Velocity, acceleration, and trajectory: To compute velocity, it is necessary to jointly process all the frames of each video and observe the positional changes that occur between frames for the same vegetation. However, this raises the challenge of identifying which vegetations detected in a given frame correspond to those detected in the subsequent frame. This problem is addressed using a family of algorithms known as tracking algorithms, which take a pair of consecutive frames and determine whether an object of interest identified in the initial frame appears in the final frame, along with the displacement it has undergone. Each point in the trajectory is determined based on the position of the previous point, adjusting the weight assigned to each algorithm according to the set of prior points.

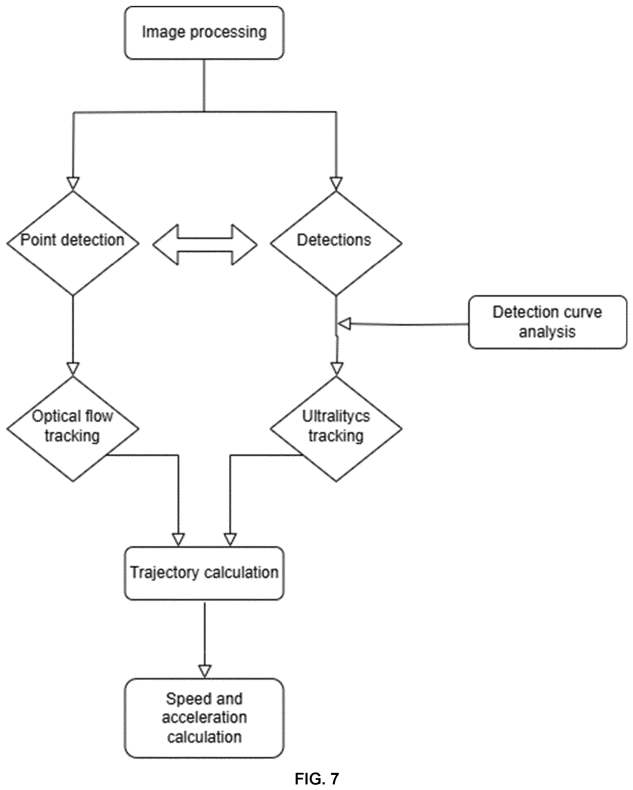

In this case, a Simple Online and Real-time Tracking (SORT) algorithm was used. This algorithm applies a Kalman filter to make new predictions and compares them with the detections predicted in the following frames based on the Intersection over Union (IOU). Each prediction is associated with a detection in such a way that the association cost (typically inversely proportional to the IOU) is minimized. The Hungarian algorithm is employed to solve this assignment optimally. Figure 7 shows a schematic example of the velocity and acceleration calculation step.

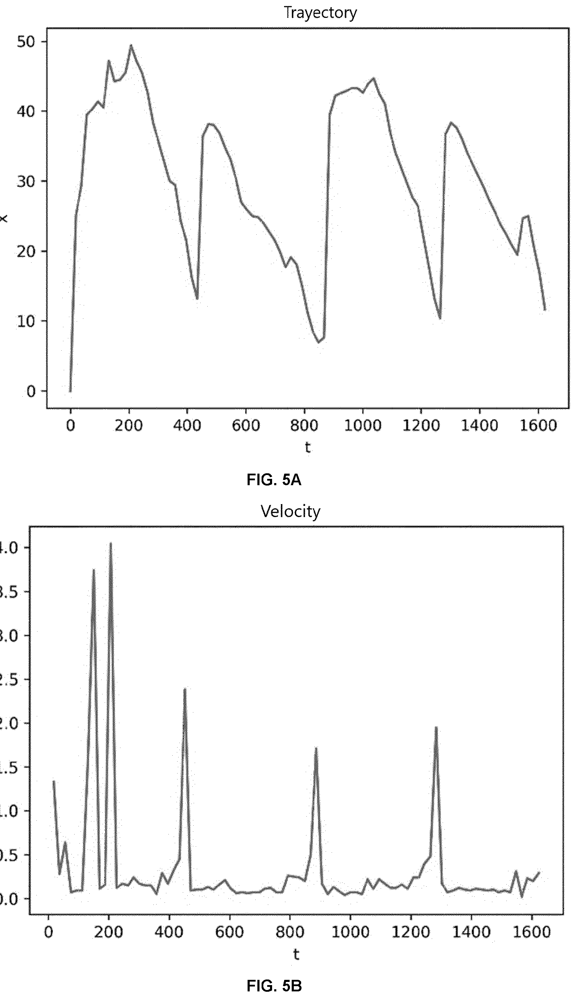

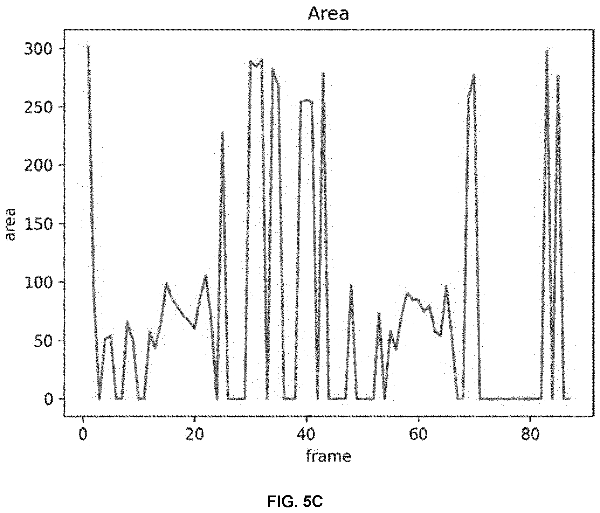

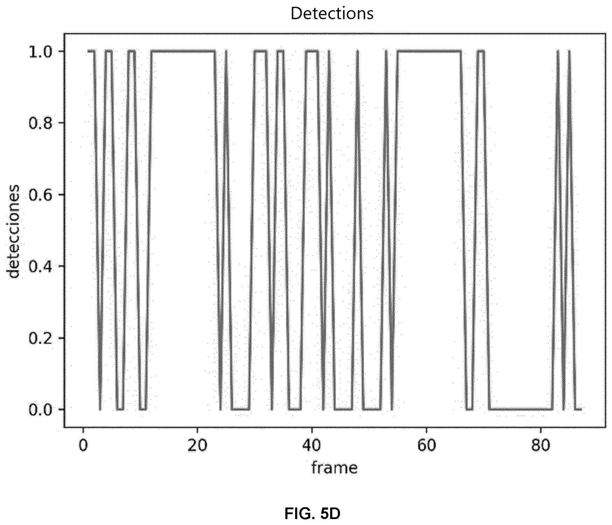

Figures 5A, 5B, 5C, and 5D show examples of the curves obtained for each of the parameters extracted using the method of the invention.

The extraction of these parameters is crucial for determining the probability of embolism. It has been demonstrated that parameters such as vegetation size are associated with the likelihood of embolism, and that the vegetation's location plays an important role in embolic risk - with mitral valve vegetations being more prone to embolism than aortic ones. Some studies have reported higher rates of systemic embolism in filamentous vegetations and those with a clustered morphology. Moreover, specific microorganisms, particularly S. aureus - with an embolic incidence ranging from 12% to 35% depending on the study series - as well as biological markers and a history of prior embolic events, have been found to be associated with embolism.

The system also comprises a correction module, configured to receive the detections and segmentations from the detection and segmentation modules, and to assess the plausibility of these detections and segmentations through machine learning algorithms and time series decomposition models.

Thus, the correction module enables the system to determine whether a detected object is, in fact, a vegetation.

As previously explained, the detection module performs a similar filtering process based on the confidence parameter, although this filtering relies solely on information from an isolated frame.

The correction module uses as input the parameter curves obtained by the parameter extraction module, in order to determine whether the parameter curves correspond to the typical behaviour of a vegetation. For this purpose, artificial intelligence algorithms aimed at detecting pattern deviations in imbalanced datasets are used. These algorithms can include both machine learning (ML) models and autoencoders.

The system may also include a valve monitoring module, which employs a third neural network (in this case, using a YOLO-based architecture) to track the motion of the valves. This neural network is similar to the one used in the detection module, but has been trained with labels defined by oriented bounding boxes (OBB) aligned with the direction of the corresponding valve, and using images from patients both with and without vegetations.

The analysed and corrected detections and segmentations are sent to a visualization module configured to generate a video similar to the input one, but with the vegetations marked and segmented, and to display the calculated results for vegetation trajectory, acceleration, area, and velocity.

In one embodiment of the present invention, a database was created comprising 244 patients who were prospectively diagnosed with endocarditis, all of whom presented vegetations on echocardiography. Clinical data was obtained structured into three main sections: baseline characteristics, clinical evolution, and echocardiographic imaging.

Echocardiographic videos were obtained from each patient and reviewed by a cardiologist specialized in imaging, who verified the presence of vegetations. To ensure the quality and relevance of the dataset, videos in which vegetations were not clearly visible, as well as low-quality videos with significant noise, artifacts, low resolution, or fewer than 10 frames, were excluded. Additionally, the videos were processed to remove any information outside the echocardiographic cone, as previously described. To improve the model's accuracy, images from patients without vegetations were also included in the training dataset as background samples.

Subsequently, a precise labelling stage was carried out for the vegetations within each echocardiographic video using a Visual Object Tagging Tool (VoTT)-type application.

Each frame of the echocardiographic videos was examined, and the vegetations were marked using bounding boxes. Additionally, the labeled data were reviewed to confirm that all vegetations were annotated accurately and consistently.

The annotated data were randomly divided into training and validation sets, ensuring that no images from the same patient were present in both groups.

Furthermore, data augmentation techniques were applied to the training set, including rotation, scaling, and flipping, to enhance variability and robustness.

Next, four different splits of patients between the training and validation groups were performed. For each split, three to four models were trained in order to select the best-performing one. Each training session lasted approximately 70 minutes.

The neural network architecture used was YOLOv11, selected for its efficiency and high performance in real-time object detection tasks. Unlike pre-trained models, this model was trained from scratch to ensure it could learn the unique features of vegetations in echocardiographic images, which differ significantly from the general objects found in standard datasets.

To train the model, the AdamW optimizer was employed, with learning rate and momentum automatically determined, resulting in respective values of 0.002 and 0.9. The batch size was set to 32, and the input image size was 640 x 640 pixels. A total of 34 epochs were used to optimize the model's performance. During the final 14 epochs, mosaic data augmentation was disabled to stabilize the training before completion. Throughout the training process, the model's performance was monitored using the validation set, and the epoch that yielded the best results was selected for evaluation.

A third group was annotated by an imaging expert for the evaluation of the algorithm. Using this group for evaluation resulted in a more demanding testing process, as only images in which the vegetation was clearly identifiable and without ambiguity regarding its boundaries were included. This third group contained 4,932 images from 30 patients.

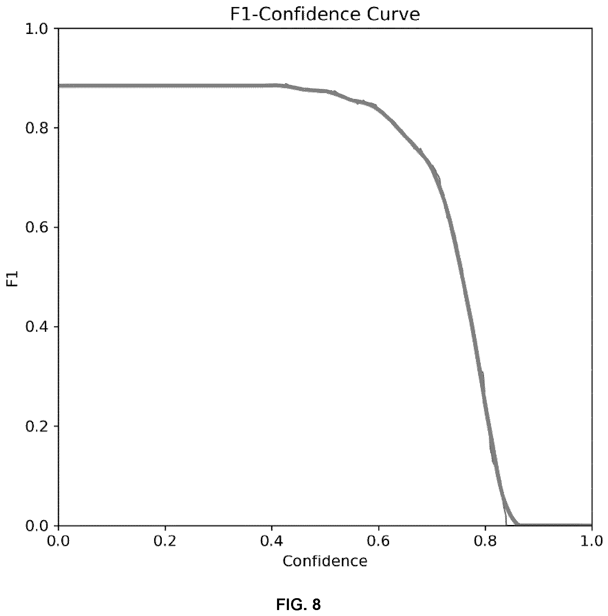

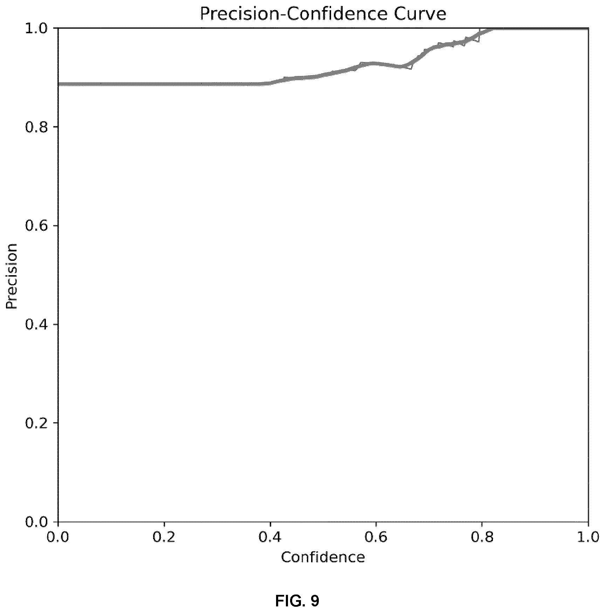

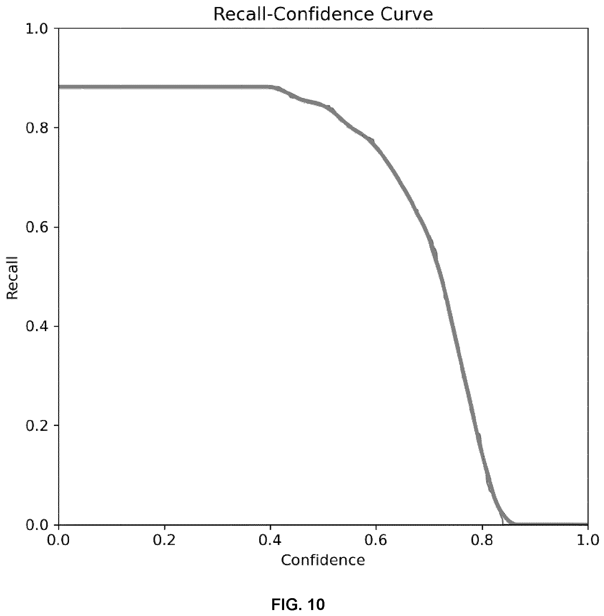

To obtain performance metrics, standard evaluation functions were used that automatically generate measurement curves commonly applied in computer vision detection algorithms:
- **F1 Score**: the harmonic mean of precision and recall, providing a balanced assessment of a model's performance by considering both false positives and false negatives. Figure 8 shows an F1 curve obtained from an example implementation of the method described in the invention.
- **Precision:** quantifies the proportion of true positives among all positive predictions, assessing the model's ability to avoid false positives. Figure 9 shows a precision curve obtained from an example implementation of the method described in the invention.
- **Recall:** calculates the proportion of true positives among all actual positives, measuring the model's ability to detect all instances of a given class. Figure 10 shows a recall curve obtained from an example implementation of the method described in the invention.

The confidence threshold for validation was set at 0.4, and the Intersection over Union (IoU) threshold for Non-Maximum Suppression (NMS) was set at 0.5.

Finally, a test was conducted to evaluate whether the neural network was capable of distinguishing between echocardiograms from patients with vegetations and those from patients without vegetations. The group of patients without vegetations consisted of individuals who did not suffer from infective endocarditis, and whose echocardiograms were not used in the training or validation sets. The group of patients with vegetations consisted of individuals diagnosed with endocarditis, in whose echocardiograms a vegetation was identified by an imaging expert.

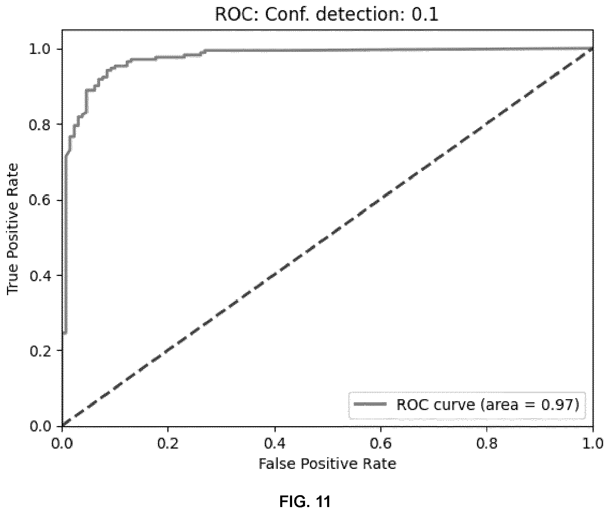

To perform this evaluation, four tests were carried out using different minimum confidence levels (0.1, 0.3, 0.5, 0.7) for detection, and the proportion of frames in which vegetation was detected was recorded, where the 'ground truth' corresponds to the group classification of the patient from whom the vegetation was obtained. Based on these results, a ROC curve was generated for each of the four tests, as shown in Figures 11 through 14, where the threshold corresponds to the ratio of frames in which vegetation was detected.

The results obtained from the model that demonstrated the best performance, and which will be used in clinical trials, are presented below.

### Internal Test Results:

- **F1 Score:** The F1 curve reached a high maximum value, achieving 0.77 at a confidence threshold of 0.345.
- **Precision:** Precision values exceeded 73% for confidence thresholds greater than 0.4.

### External Test Results:

- **F1 Score**: The F1 curve reached a high maximum value, achieving 0.73 at a confidence threshold of 0.407.
- **Precision:** Precision values exceeded 72% for confidence thresholds greater than 0.4.

The results of this study demonstrate the effectiveness of the system and method described in the invention for the automated detection of vegetations in echocardiographic images. The algorithm achieved high precision, sensitivity, and specificity in the identification of vegetations, outperforming traditional manual detection methods, which are often subject to significant operator variability.

## Claims

1. A system for detecting vegetations inside the heart through analysis of echocardiographic images, comprising:
▪ a detection module configured to receive echocardiographic images and detect one or more vegetations in each echocardiographic image using a deep neural network trained with labelled echocardiographic images; and
▪ a segmentation module connected to the detection module and configured to receive one or more echocardiographic images in which one or more vegetations detected by the detection module are present, and configured to determine the contour of the vegetations in the echocardiographic images.

2. The system for detecting vegetations according to claim 1, further comprising a parameter extraction module connected to the segmentation module and configured to receive one or more segmented echocardiographic images from the segmentation module and to analyse sequential echocardiographic images to determine parameters of shape, size, position, and movement of the vegetations.

3. The system for detecting vegetations according to any of the preceding claims, further comprising a diagnostic module configured to provide a diagnostic value associated with the risk of embolism or death based on the determined position and movement parameters of the vegetations.

4. The system for detecting vegetations according to any of the preceding claims, wherein the preprocessing module is further configured to apply to each echocardiographic image the steps of:
▪ extracting numerical values of the intensity of each pixel;
▪ converting the echocardiographic image to grayscale;
▪ delimiting an echocardiographic cone; and
▪ setting to zero all pixels outside the echocardiographic cone.

5. The system for detecting vegetations according to any of the preceding claims, wherein the detection module is further configured to detect heart structures, and the segmentation module is configured to determine an overlap region between the vegetations and the internal structures of the heart.

6. The system for detecting vegetations according to any of the preceding claims, wherein the deep neural networks comprise one or more convolutional filters (CNN) arranged in a pyramidal structure and Cross Stage Partial (CSP) blocks.

7. The system for detecting vegetations according to any of the preceding claims, wherein the deep neural networks of the segmentation module are configured to output a set of tuples corresponding to the location of contour points of the vegetation.

8. The system for detecting vegetations according to any of the preceding claims, further comprising a visualization module configured to generate a visual representation of the segmented vegetations and/or the calculated parameters.

9. The system for detecting vegetations according to any of the preceding claims, wherein the segmentation module is configured to determine a bounding box framing each vegetation, determining its centre and its dimensions.

10. The system for detecting vegetations according to any of claims 2 to 9, wherein the parameter extraction module is configured to calculate the area of the vegetation in each frame by counting a number of pixels occupied by the segmented vegetation and multiplying the number of pixels by the area of each pixel obtained using metadata from the echocardiographic image.

11. The system for detecting vegetations according to any of the claims 2 to 9, wherein the parameter extraction module is configured to calculate the speed and trajectory of the vegetations by comparing multiple consecutive frames and using a tracking algorithm.

12. The system for detecting vegetations according to any of the preceding claims, further comprising a correction module configured to apply time series decomposition models and/or artificial intelligence pattern analysis algorithms to analyse the plausibility of detections and segmentations by comparing the obtained parameters with the same parameters from previously determined vegetations.

13. The system for detecting vegetations according to any of the preceding claims, further comprising a valve monitoring module configured to determine the presence of valves in the echocardiographic images using neural networks trained with labelled valve images.

14. A method for detecting vegetations inside the heart, using the vegetation detection system according to any of claims 1 to 13, comprising the steps of:
- providing one or more echocardiographic images of the inside of the heart;
- detecting one or more vegetations in each echocardiographic image using the detection module, using the trained deep neural network;
- segmenting each of the echocardiographic images in which vegetations have been detected, to determine a contour of the vegetations using the segmentation module; and
- extracting position and motion parameters from the segmented images using the parameter extraction module.

15. A method for detecting vegetations according to claim 14, further comprising a preprocessing step including:
- extracting numerical values from the pixel intensity of each image;
- converting the echocardiographic image to grayscale;
- delimiting an echocardiographic cone; and
- setting all pixels outside the echocardiographic cone to zero.
